# EUROPEAN PATENT SPECIFICATION

(11) **EP 1 472 270 B1**
(45) Date of publication and mention of the grant of the patent: **28.12.2016**
(21) Application number: 03734819.0
(22) Date of filing: 30.01.2003
(51) Int. Cl.: C07J 9/00, C11B 7/00, C11C 3/00, C12P 7/64, A23D 9/00

(54) **FRACTIONATION OF PHYTOSTEROL ESTERS IN OIL**
FRAKTIONIERUNG VON PHYTOSTEROLESTERN IN ÖL
FRACTIONNEMENT D'ESTERS DE PHYTOSTEROLS DANS DE L'HUILE

(30) Priority: 31.01.2002 IL 14794202
(43) Date of publication of application: 03.11.2004
(73) Proprietor: Enzymotec Ltd., 36584 Kfar Baruch (IL)
(72) Inventor: GAKO-GOLAN, Einav, 20100 Karmiel (IL); BASHEER, Sobhi, 20173 Sakhnine (IL); PLAT, Dorit, 17906 Shimshit (IL); BEN-DROR, Gai, 30835 ISRAEL (IL); FARKASH, Orly, 17906 Shimshit (IL); HOTAM, Elzaphan, 30900 Zichron Yakov (IL); GOLDSHMIT, Zohar, 19125 (IL)
(74) Representative: Guerre, Dominique
(86) International application number: PCT/IL2003/000081
(87) International publication number: WO 2003/064444

(56) References cited:
- EP-A- 0 195 311
- WO-A-00/73407
- WO-A-01/72136
- WO-A-01/75083
- WO-A-99/56558
- DATABASE WPI Section Ch, Week 200166 Derwent Publications Ltd., London, GB; Class D13, AN 2001-587540 XP002242960 & KR 2001 035 226 A (ONBIO CORP), 7 May 2001 (2001-05-07)

## Description

### Field of the Invention

The present invention relates to compositions of matter which comprise diacylglycerol(s) (DAG), mainly 1,3-diacylglycerol(s), and phytosterol and/or phytostanol ester(s) (PSE) and optionally monoglycerides, dissolved or dispersed in edible fish oil, to their preparation and various uses, particularly as dietary nutrients, as food supplements and/or as ingredients in the food industry.

More specifically, the present invention relates to products of alcoholysis, esterification and/or interesterification of phytosterol(s) and/or phytostanol(s) in oil using an immobilized, optionally surfactant-coated, lipase. In particular, the invention relates to new mixtures of phytosterol and/or phytostanol ester(s) in edible fish oil. Such mixtures usually comprise an oily fraction that contains, *inter alia,* diacylglycerol(s) and phytosterol and/or phytostanol ester(s) of mainly unsaturated fatty acids, and a paste-like fraction that contains diacylglycerol(s) and phytosterol and/or phytostanol ester(s) of mainly saturated fatty acids. The present invention further provides a variety of novel, enriched mixtures of diglyceride(s) and phytosterol and/or phytostanol ester(s) in fish oil, obtained by further fractionation of the mixtures of the invention. The present invention further relates to highly purified phytosterol and/or phytostanol ester(s) and mixtures of esters obtained by the said fractionation process.

### Background of the Invention

The term "phytosterols" covers plant sterols and plant stanols. Plant sterols are naturally occurring substances present in the diet as minor components of vegetable oils. Plant sterols have a role in plants similar to that of cholesterol in mammals, e.g. forming cell membrane structures. In human nutrition, both free plant sterols and free plant stanols are beneficiary.

Phytosterols and phytosterol esters are currently being used in three main industrial categories, namely pharmaceuticals, cosmetics and alimentary products. In human nutrition, phytosterols have been shown to reduce serum cholesterol and thus reduce the risk of cardiovascular diseases (CVD) [Pollak, O.J. (1953) Circulation, 7, 702-706]. Since phytosterols are not fat-soluble, esterification processes were developed for producing fat-soluble phytosterol esters. Available common foods that contain phytosterol esters are mainly oils, margarines and to some extent dairy products. Many other current food products contain phytosterol esters, *inter alia,* pasta and chocolate products, to name but few. Incorporation of phytosterol esters in nutritional products appears to be of significance, and easily provides dietary alternatives for serum cholesterol reduction preventive therapy in a normal population in the case of preliminary cholesterolemia and, in combination with statins, in severe hypercholesterolemia.

### Diglycerides (DAG)

Diglycerides contain only two fatty acids per molecule of fat, instead of three. The major part of the two fatty acids are located mainly on each end of the fat molecule (generally referred as 1,3 positions). In nutrition, this composition of diglycerides with the unique position of fatty acids is of significant difference. The body digests DAG oil same as a conventional oil. However, one of the digestion components is different, due to the original position of the two fatty acids on the DAG oil. The gut does not prefer to reassemble fat-rich particles using this digestion component, and this is reflected by a lower number of fat-rich particles in the blood following a meal containing DAG oil. As a result, the digestion component is completely disassembled in the gut. This difference leads the body to use this portion of the fatty acids for energy rather than fat storage following absorption. Further study of DAG oil in humans showed that following consumption of as little as 20 grams of DAG oil, the number of fat-rich particles in the blood was observed to decrease compared to conventional oil (Taguchi, H., et al., J Am Coll Nutr (2000 Nov-Dec) 19(6):789-96). The level of fat-rich particles following DAG oil consumption is approximately 50% less than conventional oil at the peak level of fat-rich particles after a meal. As researchers understand the impact of post-meal fat levels on blood vessel health, this aspect of DAG oil could be even more beneficial. In people with high, particularly excess body weight and body mass index (BMI), long-term consumption of DAG oil may affect body weight and body fat changes. Initial studies indicate that body weight and body fat losses may occur to a greater extent when consuming 10-25 grams of DAG oil per day in place of conventional oil (Nagao, T., et al., J Nutr (2000) 130(4):792-7). DAG oil has been approved by the Japanese government for specific health uses pertaining to post-meal blood lipids and body weight. Additionally, a professional association of Japanese physicians has officially recommended DAG oil as part of a healthy diet. In the United States, DAG oil is generally recognized as safe (GRAS) by an expert panel review and can be marketed as a cooking oil and spread. Initial study indicates that body weight and total fat mass changes may respond in the same beneficial manner in overweight adult American individuals (FASEB J (2001) 15(4):A301) as with Japanese individuals.

WO01/32035 teaches olive oil-based products, based on especially higher grades of olive oils (such as virgin olive oils), comprising plant stanol esters and/or plant sterol esters.

US Patent No. 5,843,499 discloses oil extractable from corn fiber that contains ferulate esters (phytosterol esters which are esterified to ferulic acid), in particular sitostanyl ester, which has been shown to have cholesterol-lowering activity. It is mentioned that corn fiber oil (containing about 73% fat (triacylglycerol), 8% sterol (fatty acyl) esters, 4% free sterols, 6% diacylglycerols and 6% ferulate (sterol esters) is used as an additive to supplementary food for reducing cholesterol level.

US Patent No. 6,326,050 discloses a composition consisting of oil or fat, a diacylglycerol, a free phytosterol and tocopherol, dissolved or dispersed in the oil or fat.

None of the above mentioned publications have described a combination of phytosterol ester and diacylglycerol in oil and/or fat.

Furthermore, the prior art does not describe the enrichment of any naturally occurring oil or fat with diglycerides and phytosterol or phytostanol esters.

WO01/75083 describes a process for selective alcoholysis or esterification (interesterification) of free sterols (including phytosterols) in a fat-based product, using an immobilized, optionally surfactant-coated, lipase. The reaction takes place in the presence of triglycerides contained in the fat-based product, optionally following addition of at least one carboxylic fatty acid or ester derivative thereof. As defined in this publication, "selective alcoholysis" means that the process causes alcoholysis or esterification of the free sterol, without causing a significant change in the identity or positional distribution of the fatty acyl groups on the glycerol backbone of the triglycerides present in the oil- or fat-based product, e.g. butterfat. This publication describes modified butterfat compositions and low cholesterol food preparations obtained by the described process, and further describes the *in situ* obtained fat-based products enriched with phytosterol esters. However, WO 01/75083 does not provide any details of the composition and constituents of the products obtained by the said selective esterification reaction or their physical properties, neither does it teach or suggest specific enrichment steps of the products obtained, for improved uses of the obtained combinations of diacylglycerol(s) and phytosterol and/or phytostanol ester(s) or the corresponding enriched products.

WO 00/73407 A relates to vegetal sterol-containing fat compositions and process for producing same. These compositions are capable of providing a cholesterol absorption inhibiting effect. The compositions disclosed in WO 00/73407 comprise a plant sterol fatty acid ester and from 10 to 70% by weight of a partial glyceride.

In view of the above, it is an object of present invention to provide an edible composition consisting of phytosterol and/or phytostanol ester(s) and diacylglycerol(s) in fish oil, which may further optionally comprises monoglycerides.

It is a further object of present invention to provide the said composition as a dietary nutritional supplement (food additive) or as an ingredient in alimentary products.

It is yet a further object of present invention to prepare the said composition by either enzymatic interesterification of phytosterol(s) and/or phytostanol(s), where the triglycerides contained in the fish oil serve as acyl donors, or by just mixing or blending phytosterol and/or phytostanol ester(s) and diacylglycerol(s) with the fish oil.

It is yet another object of the present invention to provide a mixture of diacylglyceride(s) and phytosterol and/or phytostanol ester(s) in fish oil, which may be further fractionated for further enrichment.

It is another object of present invention to provide a variety of enriched mixtures of diglyceride(s) and phytosterol and/or phytostanol ester(s) of saturated fatty acids.

It is yet a further object of present invention to provide a variety of enriched mixtures of diglyceride(s) and phytosterol and/or phytostanol ester(s) of unsaturated fatty acids.

It is yet another object of present invention to provide highly purified phytosterol and/or phytostanol esters.

It is a further object of the present invention to provide a process for preparing a composition consisting of phytosterol and/or phytostanol ester(s) and diglycerides, of pre-determined composition, in fish oil.

These and other objects of the invention will become apparent as the description proceeds.

### Summary of the Invention

The invention relates to a composition of matter as claimed in any one of claims 1 to 11 which comprises at least one diacylglycerol that are mainly 1,3-diacylglycerol(s) and at least one phytosterol and/or phytostanol ester dissolved or dispersed in an edible fish oil.

The composition of matter according to the invention preferably comprises from 1 to 99wt% diacylglycerol(s) and from 1 to 99wt% phytosterol and/or phytostanol ester(s) dissolved or dispersed in edible fish oil, and may further optionally comprise monoglycerides.

In particular embodiments, the composition of matter according to the invention comprises 1 to 99wt% diacyglycerols, from 1 to 99wt% phytosterol and/or phytostanol esters and from 0 to 50wt% monoglycerides and from 1 to 99wt% triacyglycerol (s). More particularly, the composition of matter according to the invention comprises from 3 to 50wt% diacyglycerols, from 7 to 48 wt % phytosterol and/or phytostanol esters and from 2 to 90wt% triacyglycerol(s).

The phytosterol esters may be selected from the group consisting of beta-sitosterol, campesterol, stigmasterol and brassicasterol esters. The phytostanol esters may be selected from the group consisting of stigmasterol, campestanol and sitostanol.

In particular embodiments, the said phytosterol and/or phytostanol esters are esters of C₁₄-C₂₂, preferably C₁₆-C₁₈ saturated or unsaturated fatty acids, particularly oleic, linoleic, linolenic, palmitic and stearic acids.

The diacylglycerol(s) are preferably 1,3-di-fatty acyl glycerol(s).

In a further embodiment, the invention relates to a dietary food supplement and alimentary products comprising the composition of matter according to the invention.

In yet a further embodiment, the invention provides a process for preparing a composition of matter according to the invention, which process comprises the step of esterifying the phytosterol(s) and/or phytostanol(s) in fish oil to give a product (referred to as Mixture A) consisting of phytosterol and/or phytostanol esters and diglycerides in fish oil , wherein said product comprises two visibly distinct fractions being an oil fraction comprising diacylglycerol(s), triacylglycerol(s) and phytosterol and/or phytostanol esters of mainly unsaturated fatty acid(s) and a paste-like fraction comprising diacylglycerol(s), triacylglycerol(s) and phytosterol and/or phytostanol esters of mainly saturated fatty acid(s).

In the process of the invention, said oil fraction contains about 10-20% by weight of phytosterol and/or phytostanol esters of mainly unsaturated fatty acid(s) and said paste-like fraction contains about 30-35% by weight of phytosterol and/or phytostanol esters of mainly saturated fatty acid(s). The phytosterol and/or phytostanol esters employed in the process of the invention are esters of C₁₄-C₂₂, preferably C₁₆-C₁₈ saturated or unsaturated fatty acids, particularly oleic, linoleic, linolenic, palmitic and stearic acids. Particular phytosterols are selected from the group consisting of beta-sitosterol, campesterol, stigmasterol, brassicasterol, and particular phytostanols are selected from the group consisting of campestanol and sitostanol.

In another embodiment, the invention relates to a process for obtaining a product containing ester(s) of phytosterols and/or phytostanols with saturated or unsaturated fatty acids, said process comprising the steps of (a) esterifying phytosterol(s) and/or phytostanols in fish oil to give a first product (referred to as Mixture A), said first product comprising two visibly distinct fractions, the first fraction being an oil fraction which comprises diacylglycerol(s), triacylyglycerol(s) and phytosterol and/or phytostanol ester(s) of mainly unsaturated fatty acid(s), and the second fraction being a paste-like fraction which comprises diacylglycerol(s), triacylyglycerol(s) and phytosterol and/or phytostanol ester(s) of mainly saturated fatty acid(s), and (b) separating the two fractions of said Mixture A, to give a first product (referred to as Mixture B) which comprises diacylglycerol(s), triacylyglycerol(s) and phytosterol and/or phytostanol ester(s) of mainly saturated fatty acids and a second product (referred to as Mixture C) which comprises diacylglycerol(s), triacylyglycerol(s) and phytosterol and/or phytostanol ester(s) of mainly unsaturated fatty acids are obtained.

The separation of the two fractions of Mixture A may be carried out by centrifugation.

The content of phytosterol and/or phytostanol esters in Mixture B may be further enriched to form a product (referred to as Mixture B1) comprising at least 50% by weight of phytosterol and/or phytostanol esters of mainly saturated fatty acids. Thus, Mixture B may be enriched by treatment with hexane, or it may be enriched by treatment with toluene, to give substantially pure phytosterol and/or phytostanol esters (at least 98% by weight) of mainly saturated fatty acids (referred to as Mixture B2).

The content of phytosterol and/or phytostanol esters in said Mixture C may be further enriched to give a product (referred to as Mixture C1) comprising at least 25% by weight of phytosterol and/or phytostanol esters of mainly unsaturated fatty acids. Thus, Mixture C may be enriched by treatment with acetone, or by treatment with toluene, to give pure phytosterol and/or phytostanol esters (at least 95% by weight) of mainly unsaturated fatty acids (referred to as Mixture C2).

Still further, the invention relates to a dietary nutrient, alimentary product or food supplement as claimed in claim 12 or claims 24 to 26, which comprises diacylglycerol(s), triacylyglycerol(s) and phytosterol and/or phytostanol ester(s) of mainly unsaturated fatty acid(s) and/or of mainly saturated fatty acid(s). The dietary nutrient, alimentary product or food supplement, may further comprise monoglycerides.

The invention also relates to said dietary nutrient, alimentary product or food supplement, in dosage unit form, preferably a tablet or capsule.

The dietary nutrient or alimentary product according to the invention may be any one of low-cholesterol butter, cocoa butter, anhydrous milk fat, ice cream, coffee whitener and cream, dairy product, particularly cheese and other cholesterol-containing foods.

### Brief Description of the Drawings

The present invention will be more clearly understood from the detailed description of the preferred embodiments and from the attached drawings in which:
Fig. 1 represents gas chromatogram of Mixture B obtained by alcoholysis of stigmasterol in canola oil.
Fig. 2 represents gas chromatogram of Mixture C obtained by alcoholysis of stigmasterol in canola oil.
Fig. 3 represents gas chromatogram of Mixture A, taken at 60°C, following esterification of phytosterols in sunflower oil.
Fig. 4 represents gas chromatogram of Mixture B which was obtained from Mixture A of Fig. 3.
Fig. 5 represents gas chromatogram of Mixture C which was obtained from Mixture A of Fig. 3.
Fig. 6 represents gas chromatogram of Mixture C1 which was obtained from Mixture C of Fig. 5.
Fig. 7 represents gas chromatogram of Mixture C2 which was obtained from Mixture C of Fig. 5.
Fig. 8 represents gas chromatogram of Mixture B1 which was obtained from Mixture B of Fig. 3.
Fig. 9 represents gas chromatogram of Mixture B2 which was obtained from Mixture B of Fig. 3.

### Detailed Description of the Invention

In search for a still more beneficiary food supplement, the inventors have surprisingly arrived at a combination of diacyl glycerol(s), mainly 1,3-diacyl glycerol(s) (DAG) and phytosterol and/or phytostanol ester(s) (PSE) in fish oil, which is an object of the present invention. While various combinations of phytosterols and diglycerides were known (e.g. said US Patent 6,326,050), the combination of phytosterol ester(s) and diacylglycerol(s) dissolved or dispersed in fish oil is novel, and was unexpectedly found to have several advantages over known combinations of diacylglycerol(s) and free phytosterol. Thus, using phytosterol esters instead of the conventional free phytosterols, it is easier to increase their concentration in the mixture. Additionally, crystalline plant sterols do not to a significant degree dissolve in the micelli phase in the alimentary canal, and are therefore not capable of efficiently inhibiting cholesterol absorption. Moreover, fish oil is only to a limited degree capable of dissolving free sterols. Only in a dissolved form do sterols inhibit the absorption of cholesterol.

Thus, the main feature of the present invention is a composition consisting essentially of diacylglycerol(s) and phytosterol and/or phytostanol ester(s) dissolved or dispersed in edible fish oil. The composition of the present invention may be useful as a dietary nutrient, as a food supplement and/or as an ingredient in the food industry.

The compositions of the invention may be obtained, e.g., by using the selective alcoholysis or (inter)-esterification process described in WO01/75083. It has now been found that the oil product which may be obtained by the selective phytosterol esterification as described in WO 01/75083 or a similar process, consists of two fractions, each of which contains diglyceride(s) and a different type of phytosterol and/or phytostanol ester(s). More particularly, the selective alcoholysis or interesterification product is a mixture constituted of two fractions (oil and paste), wherein the phytosterol and/or phytostanol ester(s) of saturated fatty acids are substantially contained in one fraction, while esters of unsaturated fatty acids are substantially contained in the other fraction.

Thus, the present invention further provides a process for preparing said composition, which process comprises an esterification step of phytosterol(s) and/or phytostanol(s) in fish oil, to produce a product (referred to as Mixture A) consisting of phytosterol and/or phytostanol esters and diglycerides in said fish oil. This product comprises two visibly distinct fractions (layers), namely an oil fraction which contains diglycerides, triglycerides and phytosterol and/or phytostanol esters of mainly unsaturated fatty acid(s), and a paste-like fraction (spread) which contains diglycerides, triglycerides and phytosterol and/or phytostanol esters of mainly saturated fatty acid(s). The two fractions are separated to give one product (referred to as Mixture B) which comprises diglycerides, triglycerides and phytosterol and/or phytostanol esters of mainly saturated fatty acids and a second product (referred to as Mixture C) which comprises diglycerides, triglycerides and phytosterol and/or phytostanol esters of mainly unsaturated.

Further according to the invention, the content of phytosterol and/or phytostanol esters in Mixtures B and C may optionally further enriched. Importantly, the present invention enables the enrichment of any naturally occurring oil with sterol-esters and diacylglycerols.

The said Mixtures B and C can be subjected to further enrichment steps of the phytosterol and/or phytostanol esters contained in each layer.

In a further embodiment, the fractionation process of the invention comprises the enrichment of said Mixture A, to give varied new mixtures, as follows:
(i) a paste comprising about 3-50wt% diglycerides and about 7-48% by weight of phytosterol and/or phytostanol esters consisting mainly of saturated fatty acid esters, preferably C₁₄-C₂₂ saturated fatty acids, and particularly C₁₆-C₁₈ saturated fatty acid esters (herein referred to as Mixture B);
(ii) an oil comprising about 3-50wt% diglycerides and about 7-48% by weight of phytosterol and/or phytostanol esters consisting mainly of unsaturated fatty acid esters, preferably C₁₄-C₂₂ unsaturated fatty acids, and particularly C₁₆-C₁₈ unsaturated fatty acid esters (herein referred to as Mixture C);
(iii) a solid comprising about 60% by weight of phytosterol esters consisting mainly of saturated fatty acid esters, preferably C₁₄-C₂₂ saturated fatty acids, and particularly C₁₆-C₁₈ saturated fatty acid esters (herein referred to as Mixture B1);
(iv) a paste comprising about 30-35% by weight of phytosterol esters consisting mainly of unsaturated fatty acid esters, preferably C₁₄-C₂₂ saturated fatty acids, and particularly C₁₆-C₁₈ saturated fatty acid esters (herein referred to as Mixture C1);
(v) highly purified phytosterol esters consisting mainly of saturated fatty acid esters, preferably C₁₄-C₂₂ saturated fatty acids, and particularly C₁₆-C₁₈ saturated fatty acid esters (herein referred to as Mixture B2); and
(vi) highly purified phytosterol esters consisting mainly of unsaturated fatty acid esters, preferably C₁₄-C₂₂ saturated fatty acids, and particularly C₁₆-C₁₈ saturated fatty acid esters (herein referred to as Mixture C2).

These varied mixtures of phytosterol esters can be useful in a wide scope of applications such as, for example, in the food industry, both as a final product such as a dietary nutrient, and as a functional ingredient in alimentary products and as a food supplement.

As mentioned, the mixtures of the invention can also be prepared by simple blending of diglyceride(s) and phytosterol or phytostanol ester(s) in edible fish oil. Thus the invention further provides various blends of diglyceride(s) and phytosterol or phytostanol ester(s) in edible fish oil. In accordance with the invention, these blends may contain 1-99wt% phytosterol or phytostanol ester(s), 1-99wt% diglycerides and no or up to 50wt% monoglycerides, preferably 3-50wt% diglycerides and 7-48wt% phytosterol or phytostanol esters.

The ability to prepare a composition in which the type of phytosterol and/or phytostanol ester(s) is pre-determined is of great importance. More specifically, according to the present invention it is possible to prepare a composition in which the phytosterol and or phytostanol ester(s) in the composition are pre-determined by adding the desired phytosterol(s) and/or phytostanol(s) to a particular fish oil during the esterification process. The separation, following the esterification process, of the oil and paste-like fractions provides a composition consisting of mainly the desired phytosterol and/or phytostanol ester(s), containing unsaturated fatty acid residues and/or a composition consisting of mainly the desired phytosterol and/or phytostanol ester(s) containing saturated fatty acid residues. The triglycerides in the oil and/or fat may serve as an internal source for fatty acyl group(s) in the phytosterol and/or phytostanol esterification process. Alternatively, or additionally, free fatty acid(s) and/or their derivatives may serve as an external source for acyl groups.

The purification and enrichment processes of the invention can also provide highly purified phytosterol and/or phytostanol esters.

The composition and nutrients of the invention may be formulated in dosage unit forms. For example, orally administrable dosage forms may be a tablet or capsule. Each dosage unit for oral administration may contain the recommended daily amount of a mixture of the invention, or parts or multiplications thereof. The recommended dose is usually determined by the attending physician or dietician, and would depend on the age, sex, weight and general condition of the prospective consumer.

For the preparation of tablets, any pharmaceutical carrier routinely used for preparing solid formulations may be used, for example, magnesium stearate, starch, terra alba, talc, gelatin, acacia, stearic acid, lactose and sucrose. For the preparation of capsules, any routine encapsulation is suitable, for example using the said carriers in a hard gelatin capsule shell. Soft gelatin shell capsules may also be prepared by using any pharmaceutical carrier routinely used for preparing dispersions or suspensions, such as aqueous gums, celluloses, silicates or oils, which can be incorporated in a soft gelatin capsule shell.

It is to be noted that in the description herein the terms diglycerides and diacylglycerols, are used interchangingly, and so are the terms phase and fraction, and paste, paste-like and spread.

Disclosed and described, it is to be understood that this invention is not limited to the particular examples, process steps, and materials disclosed herein as such process steps and materials may vary somewhat. It is also to be understood that the terminology used herein is used for the purpose of describing particular embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The following Examples are representative of techniques employed by the inventors in carrying out aspects of the present invention. It should be appreciated that while these techniques are exemplary of preferred embodiments for the practice of the invention, those of skill in the art, in light of the present disclosure, will recognize that numerous modifications can be made without departing from the intended scope of the invention.

### Examples

### Methods

### (1) Preparation of Products: Mixture A and Fractions B and C

Mixture A is defined as the mixture obtained following either direct esterification and/or alcoholysis + interesterification of phytosterols and/or phytostanols in oil in the presence of either free fatty acids and/or the corresponding mono-, di- or tri-glycerides. For example:
Reaction is carried out batch wise. Surfactant-coated immobilized lipase and phytosterols or plant stanols are added to the oil or fat. Typical mixture contains: 1-10 parts of oil for 1 part of catalyst and 1-10 parts of oil for 1 part of phytosterol (or plant stanol). The reaction mixture is then mixed and heated to 35-70°C, preferably 50-60°C for 5-96hrs, preferably 10-48hrs. Conversion of phytosterols or phytostanols to phytosterol esters or phytostanol esters is determined by gas chromotograph. Weighed samples of reaction mixture (without catalyst) are taken from batch and diluted in n-hexane or toluene to concentrations of 20-60mg/ml and injected directly to the GC according to method described below. Concentration of free phytosterols or phytostanols is determined quantitatively (according to calibration curves) and conversion is calculated relatively to initial concentrations of phytosterols. The reaction is usually stopped at conversion of 70-100%, typically 85-95%.

Mixture A will, therefore, contain phytosterol esters and the residue of free phytosterols that did not undergo esterification.

In addition, mixture A resulting from reaction between phytosterols and natural fat or oil will contain diglycerides, monoglycerides and some concentration of free fatty acids. Enrichment in diglycerides and monoglycerides is achieved through the "donation" of fatty acids from triglycerides to the phytosterol esters formation.

At the end of the reaction the catalyst is removed from the oil by hot filtration at reaction temperature. After cooling to room temperature or bellow, the obtained Mixture A forms a solid liquid slurry with two visibly distinct and separate phases (also referred to as fractions):
Mixture B: a paste-like phase; and
Mixture C: an oil phase.

The two phases are distinct by the type of phytosterol esters they contain. The oil phase (fraction C) is enriched with esters of unsaturated fatty acids, such as, for example, oleic, linoleic and linolenic acids, whereas the spread phase (fraction B) is enriched with esters of saturated fatty acids, such as for example, palmitic and stearic acids.

Separation of mixture A to fraction B and fraction C can be obtained by centrifugation of mixture A for 5-60 minutes, preferably 10-30 minutes at 3,000-14,000g and 10-25°C.

Fraction B is obtained in its paste-like form as the bottom phase and fraction C is obtained as clear oil as the top phase.

Leaving fraction C at room temperature for 1-10 days may result in further residual precipitation of paste and loss of oil clarity. Clear form will be obtained by a second centrifugation or by filtration.

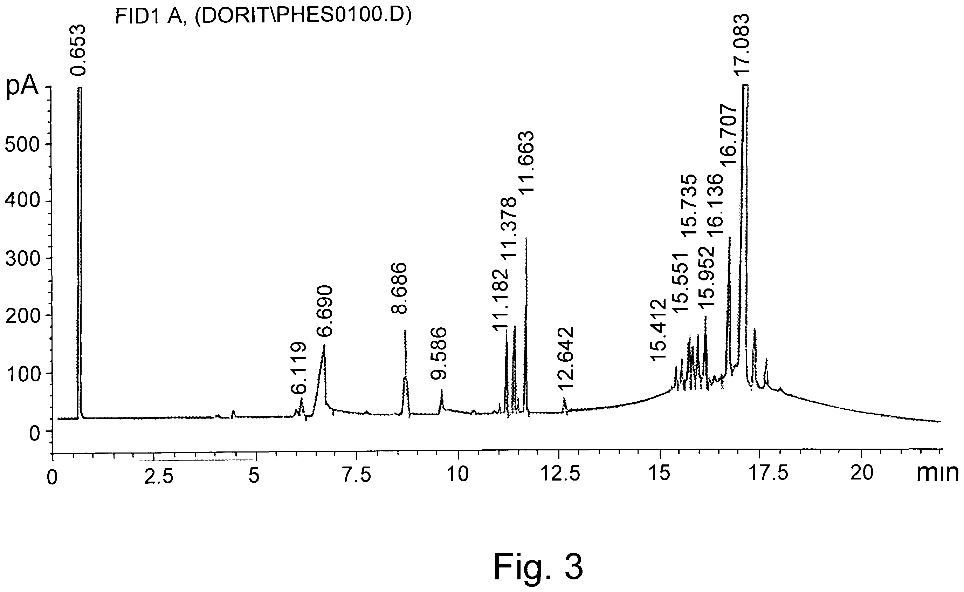

Fig. 3 represents gas chromatogram of Mixture A, form the reaction between wood phytosterols and sunflower oil taken at 60°C. The mixture comprises phytosterol esters (16% by weight) derived from sunflower oil (6 main peaks ranging from 16.136-15.413 minutes), triglycerides of the oil (17.083 and 16.707), diglycerides (12.642), phytosterols (3 main peaks, ranging from 11.663-11.182) and free fatty acids (9.586, 8.686, 6.69).

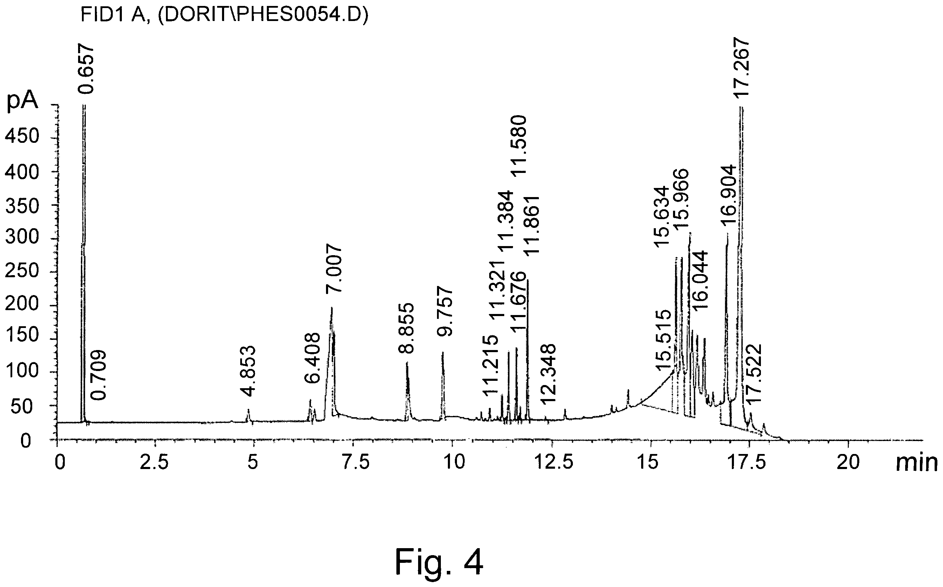

Fig. 4 represents a gas chromatogram of Mixture B. The mixture comprises phytosterol esters of saturated fatty acids (15.966, 15.751, 15.361) with a minor part of phytosterol esters of unsaturated fatty acids (16.335, 16.159, 16.044), triglycerides from the oil (16.904, 17.26), diglycerides (12.348), phytosterols (11.861, 11.580, 11.215) and minor amount of free fatty acids. The total amount of sterol esters in this mixture is 31% (w/w).

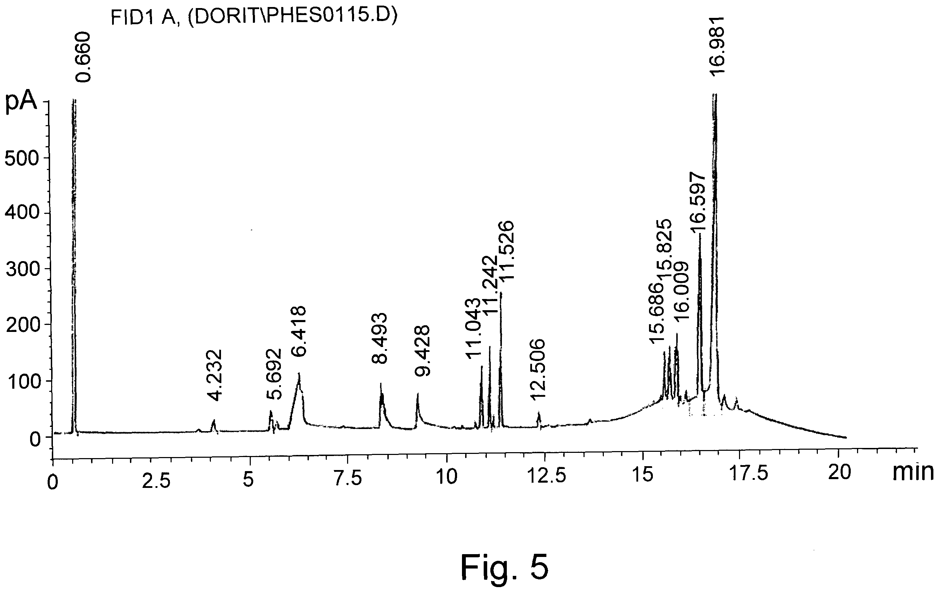

Fig. 5 represents a gas chromatogram of Mixture C. The mixture comprises phytosterol esters of unsaturated fatty acids (16.009, 15.825, 15.686), triglycerides derived from the oil (16.981, 16.597), diglycerides (12.506), phytosterols (11.526,11.242, 11.043) and minor amounts of free fatty acids. The total amount of sterol esters in this mixture is 15% (w/w).

Examples of natural oils and fats are plant and vegetable oils (linseed oil, rapeseed oil, corn oil, olive oil, cocoa butter, rice brain oil, soybean oil, palm oil, kernel oil, castor oil, cotton seed oil, coconut oil, peanut oil, sunflower oil, and the like), animal oils (butterfat, fish oil and the like).

Examples of phytosterols and phytostanols are beta-sitosterol, campesterol, stigmasterol and brassicasterol, and stigmasterol, campestanol and sitostanol.

### (2) Gas chromatography

The concentrations of phytosterols, phytostanols, phytosterol esters, phytostanol esters, fatty acids and triglycerides were determined by a gas chromatograph, HP-5890, equipped with a flame ionization detector. A capillary column, Quardex 400-1HT was used under the following separation conditions: injector and detector temperatures were maintained at 350°C, initial column temperature 160°C, followed by a 2 min. isotherm; thereafter, the oven temperature was raised at a rate of 5°C/min to 180°C; thereafter, the oven temperature was raised at a rate of 20°C/min to 400°C, followed by a 10 min. isotherm.

### (3) Further understanding of the fractionation between mixtures B and C

In order to further learn the fractionation to B and C mixtures reaction was conducted with various types of oils varying by the fatty acids compositions. For each oil source, reaction as described above is conducted and fractions B, C are separated and weighed.

Table 1 presents the weight percentages of the oily fraction (B) and the spread fraction (C) obtained from esterification reaction of phytosterols with soybean source in different oils. Reaction was conducted in shaker incubator at 50°C for 24 hours, with phytosterols:oil weight ratio of 1:9 and catalyst:oil weight ratio of 1:5.

**Table 1**

| **Mass fraction of fractions B (spread) and C (oil) obtained fron esterification of soybean phytosterols in different vegetable oils** | | | |
|---|---|---|---|
| Type of oil used in the reaction | Saturated fatty acids in edible oil (%w/w) | Oil phase (mixture C) mass fraction (%w/w) | Spread phase (mixture B) mass fraction (%w/w) |
| Pure triolein* | 0 | 100 | 0 |
| Fish oil** | 1 | 100 | 0 |
| Canola*** | 9.2 | 93 | 7 |
| Sunflower*** | 11.0 | 91.5 | 8.5 |
| Soybean*** | 15.8 | 80 | 20 |
| Olive**** | 19.8 | 76 | 24 |

| | | | |
|---|---|---|---|
| **Standard triolein purity>99%-Sigma* ***Epax 2050- "Pronova"* ****Commercial refined sources* *****Extra virgin Olive Oil* | | | |

From Table 1 it is clearly shown that the mass fraction of the spread phase (mixture B) increases with the increase of saturated fatty acids ratio in the fatty acids source: the oil in this case.

In addition, mixture A and fractions B and C are further analyzed to evaluate the differences in the composition of fatty acids in the phytosterol esters molecules.

Purification of phytosterol esters from the mixture is achieved by glass column chromatography using Merck's silica gel 60 (0.04-0.063µm) and Petroleum ether: ether mixture (2-10% of ether) as solvent. At this separation system phytosterol esters elute first and are easy to purify. Fractions containing phytosterol esters were combined and fatty acids in the pure phytosterol esters fraction, were transferred to methyl esters by applying AOC's method Ce-2-66. Separation and quantitation of methyl esters was performed on capillary gas chromatography using internal standard.

Table 2 presents the fatty acid composition of selected vegetable oils as reference to the fraction results.

Tables 3-5 present the fatty acids composition of mixtures A, B, and C mixtures resulting from reactions with different types of oils.

SFA designates saturated fatty acids, MUFA designates monounsaturated fatty acid and PUFA designates polyunsaturated fatty acids. The reaction conditions are similar to the described for Table 1.

**Table 2**

| **Fatty acid content of source vegetable oils** | | | |
|---|---|---|---|
| **Type of Oil** | Fatty Acid Composition in Oil | | |
| | SFA Wt% | MUFA Wt% | PUFA Wt% |
| Olive | 20 | 69.5 | 10.5 |
| Canola | 9 | 55 | 36 |
| Soybean | 16 | 23.5 | 60.5 |
| Sunflower | 11 | 27.5 | 61.5 |

**Table 3**

| **Fatty acid content of phytosterol esters obtained in mixture A** | | | |
|---|---|---|---|
| **Type of Oil** | Fatty acid composition of phytosterol esters | | |
| | SFA Wt% | MUFA Wt% | PUFA Wt% |
| Olive | 17 | 64 | 19 |
| Soybean | 11 | 25 | 64 |

**Table 4**

| **Fatty acid content of phytosterol esters obtained in mixture B (spread phase)** | | | |
|---|---|---|---|
| **Type of Oil** | Fatty acid composition of phytosterol esters | | |
| | SFA Wt% | MUFA Wt% | PUFA Wt% |
| Olive | 32 | 53 | 15 |
| Canola | 30 | 45 | 26 |
| Soybean | 41 | 17 | 42 |
| Sunflower | 43 | 17 | 40 |

**Table 5**

| **Fatty acid content of phytosterol esters obtained in mixture C (oil-liquid phase)** | | | |
|---|---|---|---|
| **Type of Oil** | Fatty acid composition of phytosterol esters | | |
| | SFA Wt% | MUFA Wt% | PUFA Wt% |
| Olive | 6 | 73 | 21 |
| Canola | 4 | 59 | 37 |
| Soybean | 5 | 26 | 69 |
| Sunflower | 4 | 25 | 67 |

The data presented in Tables 2-5 indicates the following:
1. Comparing Tables 2 and 3, one can see that the ratio between saturated to unsaturated fatty acids in the phytosterol esters is dependent on the fatty acid composition of the oil used in reaction. In other words one may say that (MUFA+PUFA)/SFA in mixtures A (Table 3) closely resembles the same ratio in the natural oils (Table 2).
2. Comparing Tables 4 to 5 leads to a conclusion that the main feature differentiating the phytosterol esters contained in mixtures B (spread) and C (oil) is the fatty acid composition. More specifically, the esterification reaction results in enrichment of saturated fatty acids phytosterol esters in fraction B and unsaturated fatty acids phytosterol esters in fraction C. This fractionation of the sterol esters enables the formation of two distinct phases.
3. From Table 5 it can be seen that the oily phase (fraction C) contains above 94% unsaturated fatty acids phytosterol esters, regardless of the unsaturated fatty acid composition of source oil.
4. The difference in physical form of fraction B (spread) and fraction C (liquid) is explained through the relatively high concentration of saturated fatty acids phytosterol esters in the spread phase which are responsible for the higher melting point of the spread.

### (4) Further enrichment of mixtures A, B and C

### 4.1. Enrichment of fraction A with phytosterol esters and diglycerides by molecular distillation

Further enrichment of the reaction product with diglycerides and phytosterol esters can be obtained by introducing molecular distillation. The three main components are distilled over in an order according to their molecular weight: first the diglycerides, then the phytosterol esters and then the triglycerides. Therefore, varying feed stream composition and distillation conditions can lead to a predicted, diglycerides and phytosterol esters enriched distillate and triglycerides enriched residue. Distillation conditions of 0.001-0.01mbar, preferably 0.001-0.005mbar and 200-300°C, preferably 220-280°C, enable a distillation of the 2 lighter components: diglycerides and phytosterol esters.

### 4.2 Enrichment of fractions B and C with phytosterol esters by crystallization

Due to the relatively low solubility and high melting point of phytosterol esters comparing to other components in product, dissolving fractions in various organic solvents followed by cooling stage leads to a selective crystallization of phytosterol esters. This process enables an increase in phytosterol esters concentration.

Organic solvents that can be used are hexane, toluene, acetone, ethanol, methanol and others.

### Example 1: Lipase-catalyzed alcoholysis of stigmasterol in canola oil

### 1.1 Enzymatic Reaction

The lipase-catalyzed conversion of stigma sterol to stigma sterol ester in canola oil was performed using the following reaction conditions: 9g of a lipase preparation obtained according to WO 01/75083 were added to 50ml canola oil enriched with 4gr stigmasterol. The reaction mixture was then heated to 60°C for 14 hours. Following this period, samples were taken, diluted with n-hexane (30µl reaction mixture/600µl n-hexane) and injected into a gas chromatography system, as described above. The concentration of unreacted stigmasterol was determined according to GC and conversion was calculated as 94%.

### 1.2 Separation of the reaction products

At the end of the reaction the enzyme was filtered and the mixture was cooled to 25°C and left for 12hrs.

The obtained mixture (herein referred to as Mixture A) has two distinct separated phases/fractions: an oil phase/fraction and a spread (paste-like) phase/fraction.

Mixtures B and C are obtained by centrifugation of Mixture A for 5 minutes, at 8000 x rpm at about 15°C.

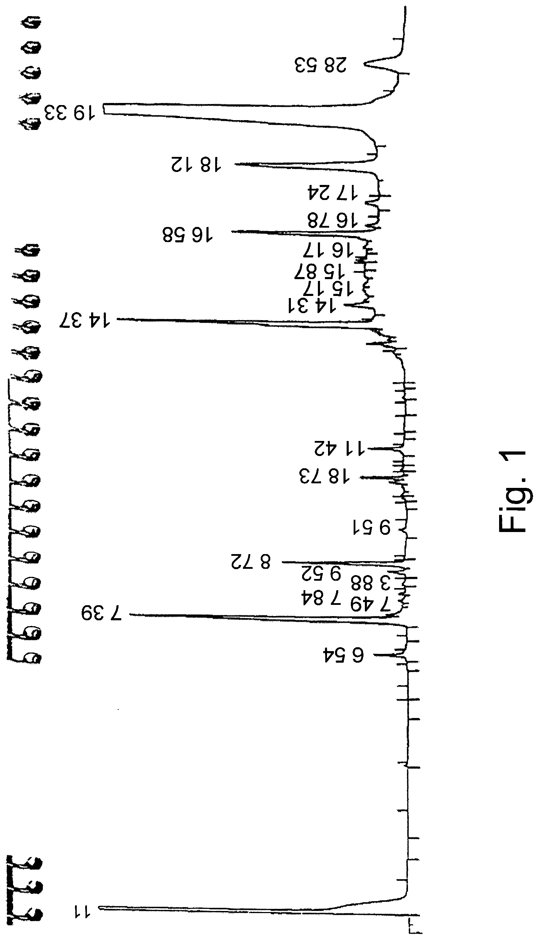

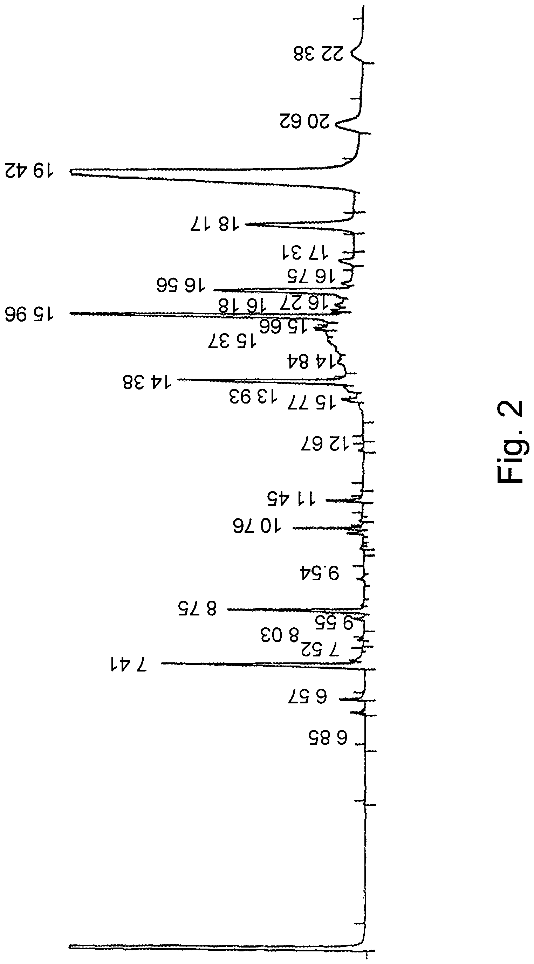

Figs. 1 and 2 demonstrate gas chromatogram analysis of both individual fractions.

Fig. 1 represents gas chromatogram of Mixture B obtained by alcoholysis of stigmasterol in canola oil. The mixture comprises stigmasterol palmitate (as confirmed by chemically synthesized analogous standard) (16.5minutes), triglycerides derived from the canola oil (19.35-18.12-minutes), diglycerides (14.37 minutes), stigmasterol (10.73 minutes) monoglycerides (11.42 minutes) and free fatty acids (8.72-6.54 minutes).

Fig. 2 represents a gas chromatogram of Mixture C obtained by alcoholysis of stigmasterol in canola oil. The mixture comprises stigmasterol oleate (as confirmed by chemically synthesized analogous standard) (15.96 minutes),stigmasterol palmitate (16.56 minutes) triglycerides from the oil (19.42-18.17), diglycerides (14.38), stigmasterol (10.76 minutes), monoglycerides (11.45 minutes) and free fatty acids (8.75-6.57 minutes).

### Example 2: Lipase-catalyzed alcoholysis of phytosterols from soybean source in olive oil

### 2.1 The enzymatic reaction

100g of a lipase preparation obtained according to WO 01/75083 and 120g of phytosterols from soybean source (containing 0.3-4% Brassicasterol, 20-30% Campasterol; 11-20% Stigmasterol; >40% Beta Sitosterol) were added to 450g extra virgin olive oil in a 1 liter glass stirred reactor. The reaction mixture is then heated to 50°C and stirred for 24 hours. Conversion of reaction was 93%. The catalyst is separated by filtration at 50°C. The filtered catalyst is added to a fresh mixture of 450g extra virgin olive oil with 120gr of unreacted phytosterols. The 2^{nd} batch reaches 93% conversion after 24hrs as well. Filtration of catalyst is repeated and 566g of product (mixture A) are left at room temperature for 20hrs.

Mixtures B and C are obtained by centrifugation of Mixture A for 15 minutes, at 7,500xg and 25°C.

Mixture B, the spread fraction, was of 164 gr weight, whereas mixture C, the oil fraction, weighed 402gr.

The composition of mixture A was: 16.3% diglycerides, 6.18% monoglycerides, 30.5% phytosterol esters and 5% free fatty acids and balance of triglycerides.

Fatty acid compositions of phytosterol esters from fractions B and C were determined as described above. Results are presented in Table 6.

**Table 6**

| **Fatty acid composition of phytosterol esters** | | |
|---|---|---|
| Fatty acid | %in Oil (fraction C) | %in spread (fraction B) |
| C16 | 3.0 | 29.9 |
| C16:1 | 1.8 | 1.2 |
| C18 | 1.2 | 2.0 |
| C18:1 | 71.5 | 51.7 |
| C18:2 | 20.9 | 14.1 |
| C18:3 | 1.4 | 0.9 |
| C20 | 0.2 | 0.1 |

### Example 3: Further Enrichment of phytosterol ester and diglycerides by molecular distillation:

A reaction mixture obtained from canola oil source containing: 13wt% phytosterol esters, 20wt% diglycerides, 0.1-1wt% of free phytosterols and 5wt% free fatty acids was fed to a molecular still (glass made, 2 inch diameter, lab unit). First pass was conducted at conditions enabling removal of free phytosterol and free fatty acids: 180-200°C and 0.01-0.001 mbar. Residue of first pass was fed again under different conditions that enable a variety of distillate compositions, as shown in Tables 7 and 8. PSE designates phytosterol esters.

**Table 7**

| **Distillate mass and phytosterol esters yield in the distillate fraction (0.001-0.005 mbar at different temperatures)** | | |
|---|---|---|
| Temp (°C) | Distillate mass/feed mass | PSE mass in distillate/PSE mass in feed |
| 235 | 0.17 | 0.40 |
| 240 | 0.23 | 0.58 |
| 250 | 0.28 | 0.62 |
| 265 | 0.53 | 0.95 |

In order to obtain above 90% of phytosterol esters in the distillate phase the necessary conditions were found to be: 260-265°C and 0.001-0.005 mbar.

**Table 8**

| **Distillate composition obtained at distillation (0.001-0.005 mbar at different temperatures)** | | | |
|---|---|---|---|
| Temp (°C) | Composition of distillate | | |
| | Phytosterol esters Wt% | Triglycerides Wt% | Diglycerides Wt% |
| 235 | 27 | 22 | 51 |
| 240 | 32 | 28 | 40 |
| 250 | 32 | 25 | 43 |
| 265 | 27 | 52 | 21 |

Calculations were done based on the feed mass balance and relative integrated area obtained from GC.

### Example 4: Further enrichment of phytosterol esters in fraction B and C by solvent crystallization

(i) For concentrating the phytosterol ester content in Mixture C, 100ml of acetone were added to 100ml Mixture C obtained as described under Example 2 above. The mixture was cooled to -20°C for 12 hours. During this period a white solid precipitation (Mixture C1) was deposited from the oily mixture. The solid precipitation (25g) was filtered in a cold filtration process and was washed few times with cold acetone.

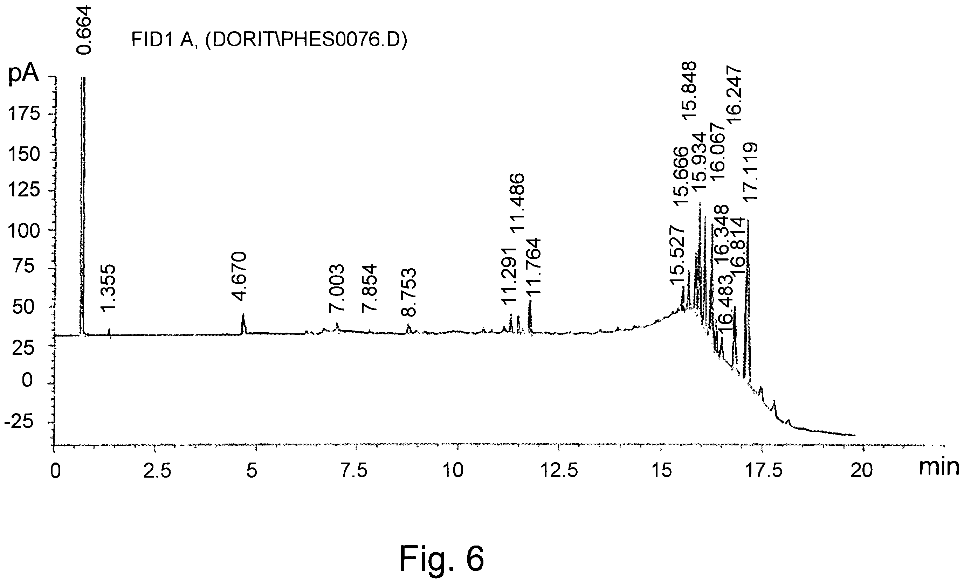

   Fig. 6 represents gas chromatogram of Mixture C1. The mixture comprises phytosterol esters of unsaturated fatty acids (16.247, 16.067, 15.934), triglycerides derived from the oil (17.119, 16.814), diglycerides, phytosterols (11.764, 11.486, 11.291) and minor amounts of free fatty acids. The total amount of sterol esters in this mixture is 30% (w/w).
(ii) For further concentrating the phytosterol ester content in Mixture C, 50ml of toluene were added to 20g solid Mixture C, obtained as described above, under mild heating. The mixture was cooled to -20°C for 12 hours. During this period a white solid precipitate (Mixture C2) was deposited from the solution. The solid precipitation (4g) was filtered in a cold filtration process and was washed few times with cold toluene.

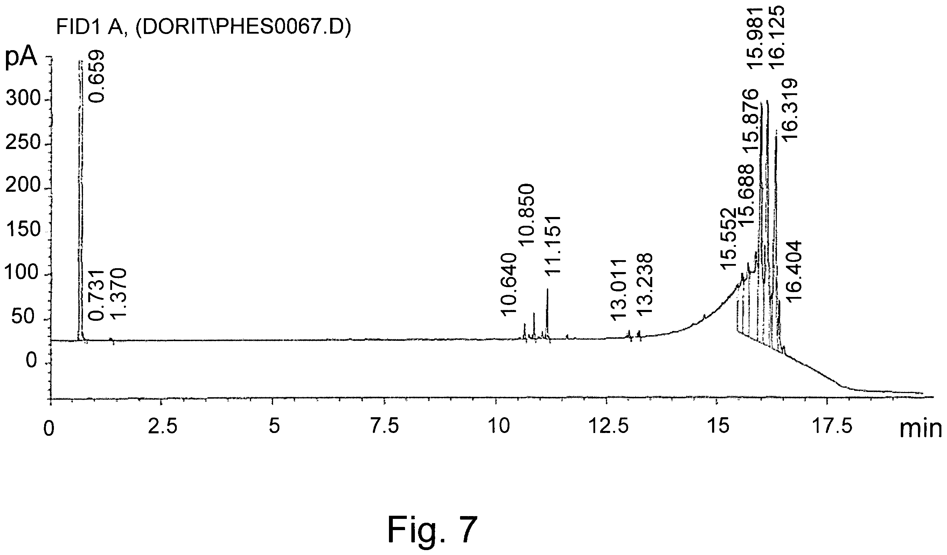

Fig. 7 represents a gas chromatogram of Mixture C2. The mixture comprises phytosterol esters of unsaturated fatty acids (16.319, 16.125, 15.981), phytosterols (11.151, 10.850, 10.640) and diglycerides (13.24, 13.01). The total amount of sterol esters in this mixture is >90% (w/w).
(i) For concentrating the phytosterol ester content in Mixture B, 100ml of hexane were added to 90g Mixture B obtained as described in Example 2. The mixture was cooled to -20°C for 12 hours. During this period a white solid precipitate (Mixture B1) was deposited from the solution. The solid precipitate (35g) was filtered in a cold filtration process and was washed few times with cold hexane.

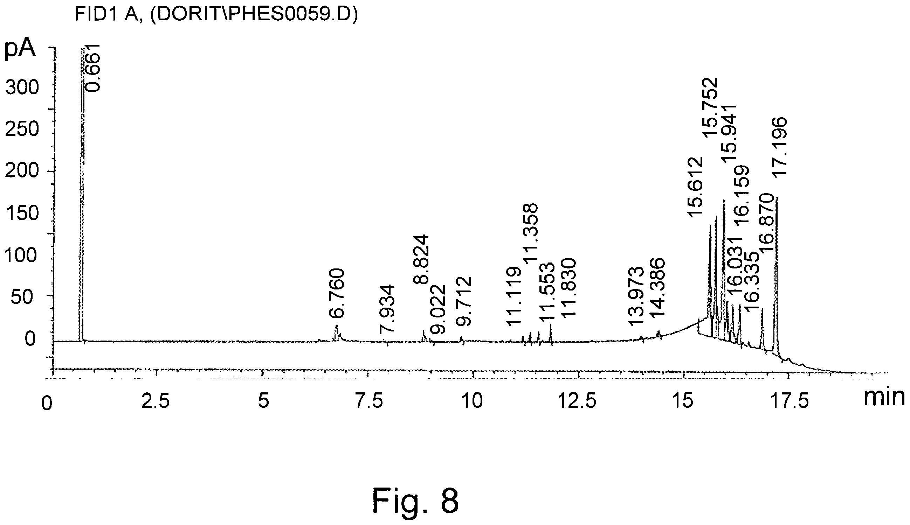

   Fig. 8 represents gas chromatogram of Mixture B1. The mixture comprises mainly phytosterol esters of saturated fatty acids (15.941, 15.752, 15.612), small amount of phytosterol esters of unsaturated fatty acids (16.335, 16.159, 16.031), triglycerides derived from the oil (17.196, 16.870), diglycerides, phytosterols (11.830, 11.553, 11.190), diglycerides (14.39, 13.97) and minor amounts of free fatty acids. The total amount of sterol esters in this mixture is 50% (w/w).
(ii) For further concentrating the phytosterol ester content in Mixture B, 50ml of toluene were added to 20g solid Mixture B, obtained as described in Example 2, under mild heating. The mixture was cooled to -20°C for 12 hours. During this period a white solid precipitate (Mixture B2) was deposited from the solution. The solid precipitate (5g) was filtered in a cold filtration process and was washed few times with cold toluene.

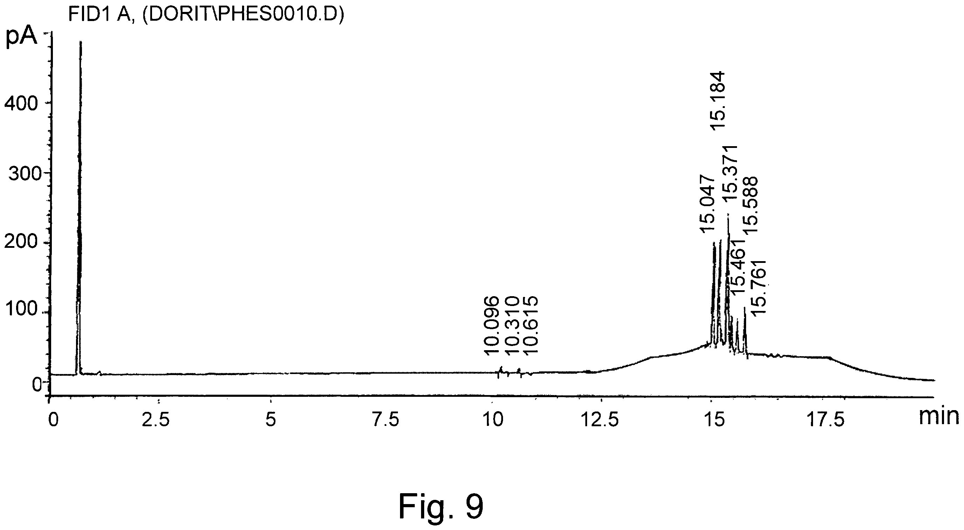

Fig. 9 represents a gas chromatogram of Mixture B2. The mixture comprises mainly phytosterol esters of saturated fatty acids (15.371, 15.184, 15.047), smaller amounts of phytosterol esters of unsaturated fatty acids (15.761, 15.588, 15.461) and phytosterols (10.615, 10.310, 10.096). The total amount of sterol esters in this mixture is >95% (w/w).

## Claims

1. A composition of matter comprising diacylglycerol(s) that are mainly 1,3-diacylglycerol(s) and at least one phytosterol ester(s) and/or phytostanol ester(s) dissolved or dispersed in fish oil.

2. A composition of matter according to claim 1, wherein said composition is separable into two visibly distinct fractions being an oil fraction comprising diacylglycerol(s) that are mainly 1,3-diacylglycerol(s), triacylglycerol(s) and phytosterol esters and/or phytostanol esters of mainly unsaturated fatty acid(s) and a paste-like fraction comprising diacylglycerol(s) that are mainly 1,3-diacylglycerol(s), triacylglycerol(s) and phytosterol esters and/or phytostanol esters of mainly saturated fatty acid(s).

3. A composition of matter according to claim 1, wherein said composition comprises two distinct fractions being an oil fraction comprising diacylglycerol(s) that are mainly 1,3-diacylglycerol(s), triacylglycerol(s) and phytosterol ester(s) and/or phytostanol ester(s) of mainly unsaturated fatty acid(s) and a paste-like fraction comprising diacylglycerol(s) that are mainly 1,3-diacylglycerol(s), triacylglycerol(s) and phytosterol ester(s) and/or phytostanol ester(s) of mainly saturated fatty acid(s).

4. A composition of matter according to any one of claims 1 to 3, comprising from 1 to 99wt% diacylglycerol(s) that are mainly 1,3-diacylglycerol(s) and from 1 to 99wt% said phytosterol ester(s) and/or phytostanol ester(s) dissolved or dispersed in fish oil.

5. A composition of matter according to any one of claims 1 to 4, further comprising monoglycerides.

6. A composition of matter according to claim 4 or claim 5, comprising from 1 to 99wt% diacylglycerol(s) that are mainly 1,3-diacylglycerols, from 1 to 99wt% phytosterol ester(s) and/or phytostanol ester(s) and from 0 to 50wt% monoglycerides and from 1 to 99wt% triacylglycerol(s), preferably said composition comprises from 3 to 50wt% diacylglycerol(s) that are mainly 1,3-diacylglycerols, from 7 to 48 wt% phytosterol ester(s) and/or phytostanol ester(s) and from 2 to 90wt% triacylglycerol(s).

7. A composition of matter according to any one of claims 1 to 6, wherein said phytosterol is selected from the group consisting of beta-sitosterol, campesterol, stigmasterol and brassicasterol, and wherein said phytostanol is selected from the group consisting of stigmastanol, campestanol and sitostanol.

8. A composition of matter according to any one of claims 1 to 7, wherein said phytosterol ester(s) and/or phytostanol esters are esters of C₁₄-C₂₂, preferably C₁₆-C₁₈ saturated or unsaturated fatty acids, as for example oleic, linoleic, linolenic, palmitic and stearic acids.

9. A composition of matter according to claim 8, wherein said phytosterol ester(s) and/or phytostanol esters are esters of C₁₄-C₂₂, preferably C₁₆-C₁₈ unsaturated fatty acids.

10. A composition of matter according to claim 1 which comprises diacylglycerol(s) that are mainly 1,3-diacylglycerol(s), triacylglycerol(s) and at least 25% by weight phytosterol ester(s) and/or phytostanol ester(s) of mainly unsaturated fatty acids.

11. A composition according to claim 10 comprising at least 95% by weight phytosterol ester(s) and/or phytostanol ester(s) of mainly unsaturated fatty acids.

12. A dietary food supplement or an alimentary product comprising the composition of matter according to any one of claims 1 to 11.

13. A process for preparing a composition of matter comprising diacylglycerol(s) that are mainly 1,3-diacylglycerol(s) and at least one phytosterol ester(s) and/or phytostanol ester(s), dissolved or dispersed in fish oil, said process comprising the step of esterifying the phytosterol(s) and/or phytostanol(s) in fish oil to give a product (referred to as Mixture A) consisting of at least one phytosterol ester and/or phytostanol ester, and diacylglycerol(s) that are mainly 1,3-di-acylglycerol(s) in fish oil.

14. A process according to claim 13, wherein said product is separable into or comprises two visibly distinct fractions, being an oil fraction comprising diacylglycerol(s) that are mainly 1,3-diacylglycerol(s), triacylglycerol(s) and phytosterol esters and/or phytostanol esters of mainly unsaturated fatty acid(s) and a paste-like fraction comprising diacylglycerol(s) that are mainly 1,3-diacylglycerol(s), triacylglycerol(s) and phytosterol esters and/or phytostanol esters of mainly saturated fatty acid(s).

15. A process according to claim 13, wherein said oil fraction contains about 10-20% by weight of phytosterol ester(s) and/or phytostanol ester(s) of mainly unsaturated fatty acid(s) and said paste-like fraction of said product contains about 30-35% by weight of phytosterol ester(s) and/or phytostanol ester(s) of mainly saturated fatty acid(s).

16. A process according to any one of claims 13 or 15, wherein said phytosterol esters and/or phytostanol esters are esters of C₁₄-C₂₂, preferably C₁₆-C₁₈ saturated or unsaturated fatty acids, wherein said fatty acids are selected from the group consisting of oleic, linoleic, linolenic, palmitic and stearic acids.

17. A process according to any one of claims 13 to 16, wherein said phytosterol is selected from the group consisting of beta-sitosterol, campesterol, stigmasterol, brassicasterol, and said phytostanol is selected from the group consisting of campestanol and sitostanol.

18. A process according to any one of claims 13 to 17, further comprising the steps of separating the two fractions of said Mixture A, to give a first product (referred to as Mixture B) which comprises diacylglycerol(s) that are mainly 1,3-diacylglycerol(s), triacylglycerol(s) and phytosterol ester(s) and/or phytostanol ester(s) of mainly saturated fatty acids and a second product (referred to as Mixture C) which comprises diacylglycerol(s) that are mainly 1,3-diacylglycerol(s), triacylglycerol(s) and phytosterol ester(s) and/or phytostanol ester(s) of mainly unsaturated fatty acids.

19. A process according to claim 18, wherein the separation of the two fractions of Mixture A is carried out by centrifugation.

20. A process according to claim 18, wherein the content of phytosterol ester(s) and/or phytostanol esters in said Mixture C is further enriched to give a product (referred to as Mixture C1) comprising at least 25% by weight of phytosterol and/or phytostanol esters of mainly unsaturated fatty acids.

21. A process according to claim 20, wherein said Mixture C is enriched by treatment with acetone.

22. A process according to claim 20, wherein said Mixture C is enriched by treatment with toluene, to give pure phytosterol and/or phytostanol esters (at least 95% by weight) of mainly unsaturated fatty acids (referred to as Mixture C2).

23. Use of a composition of matter which comprises a mixture of diacylglycerol(s) that are mainly 1,3-diacylglycerol(s) and triacylglycerol(s) with phytosterol ester(s) and/or phytostanol ester(s) of mainly unsaturated fatty acid(s) dissolved or dispersed in fish oil, as a dietary nutrient or food supplement.

24. A dietary nutrient or food supplement according to claim 12, further comprising monoacylglycerols(s).

25. A dietary nutrient or food supplement according to claim 12 or claim 24, in dosage unit form, preferably a tablet or capsule.

26. A dietary nutrient according to any one of claims 12, 24 and 25, being any one of low-cholesterol butter, cocoa butter, anhydrous milk fat, ice cream, coffee whitener and cream, dairy product, particularly cheese and other cholesterol-containing foods.

## Patentansprüche

1. Stoffzusammensetzung, umfassend Diacylglycerin(e), die hauptsächlich 1,3-Diacylglycerin(e) und mindestens ein in Fischöl gelöstes oder dispergiertes Phytosterolester und/oder Phytostanolester sind.

2. Stoffzusammensetzung nach Anspruch 1, wobei die Zusammensetzung in zwei sichtbar verschiedene Fraktionen trennbar ist, eine Ölfraktion, umfassend Diacylglycerin(e), die hauptsächlich 1,3-Diacylglycerin(e), Triacylglycerin(e) und Phystosterolester und/oder Phytostanolester von hauptsächlich ungesättigter/ungesättigten Fettsäure(n) sind, und eine pastenartige Fraktion, umfassend Diacylglycerin(e), die hauptsächlich 1,3-Diacylglycerin(e), Triacylglycerin(e) und Phystosterolester und/oder Phytostanolester von hauptsächlich gesättigter/gesättigten Fettsäure(n) sind.

3. Stoffzusammensetzung nach Anspruch 1, wobei die Zusammensetzung zwei verschiedene Fraktionen umfasst, eine Ölfraktion, umfassend Diacylglycerin(e), die hauptsächlich 1,3-Diacylglycerin(e), Triacylglycerin(e) und Phystosterolester und/oder Phytostanolester von hauptsächlich ungesättigter/ungesättigten Fettsäure(n) sind, und eine pastenartige Fraktion, umfassend Diacylglycerin(e), die hauptsächlich 1,3-Diacylglycerin(e), Triacylglycerin(e) und Phystosterolester und/oder Phytostanolester von hauptsächlich gesättigter/gesättigten Fettsäure(n) sind.

4. Stoffzusammensetzung nach einem der Ansprüche 1 bis 3, umfassend von 1 bis 99 Gew.-% Diacylglycerin(e), die hauptsächlich 1,3-Diacylglycerin(e) sind, und von 1 bis 99 Gew.-% eines oder mehrerer in Fischöl gelöster oder dispergierter Phytosterolester und/oder Phytostanolester.

5. Stoffzusammensetzung nach einem der Ansprüche 1 bis 4, ferner umfassend Monoglyceride.

6. Stoffzusammensetzung nach Anspruch 4 oder Anspruch 5, umfassend von 1 bis 99 Gew.-% Diacylglycerin(e), die hauptsächlich 1,3-Diacylglycerine sind, von 1 bis 99 Gew.-% Phytosterolester und/oder Phytostanolester und von 0 bis 50 Gew.-% Monoglyceride und von 1 bis 99 Gew.-% Triacylglycerin(e), wobei die Zusammensetzung vorzugsweise von 3 bis 50 Gew.-% Diacylglycerin(e) umfasst, die hauptsächlich 1,3-Diacylglycerine sind, von 7 bis 48 Gew.-% Phytosterolester und/oder Phytostanolester und von 2 bis 90 Gew.-% Triacylglycerin(e).

7. Stoffzusammensetzung nach einem der Ansprüche 1 bis 6, wobei das Phytosterol ausgewählt ist aus der Gruppe, bestehend aus beta-Sitosterol, Campesterol, Stigmasterol und Brassicasterol und wobei das Phytostanol ausgewählt ist aus der Gruppe, bestehend aus Stigmastanol, Campestanol und Sitostanol.

8. Stoffzusammensetzung nach einem der Ansprüche 1 bis 7, wobei das oder die Phytosterolester und/oder das oder die Phytostanolester C₁₄-C₂₂-Ester sind, vorzugsweise gesättigte oder ungesättigte C₁₆-C₁₈-Fettsäuren, z. B. Öl-, Linol-, Linolen-, Palmitin- und Stearinsäuren.

9. Stoffzusammensetzung nach Anspruch 8, wobei das oder die Phytosterolester und/oder Phytostanolester C₁₄-C₂₂-Ester sind, vorzugsweise ungesättigte C₁₆-C₁₈- Fettsäuren.

10. Stoffzusammensetzung nach Anspruch 1, umfassend Diacylglycerin(e), die hauptsächlich 1,3-Diacylglycerin(e), Triacylglycerin(e) und mindestens 25 Gew.-% Phytosterolester und/oder Phytostanolester aus hauptsächlich ungesättigten Fettsäuren sind.

11. Zusammensetzung nach Anspruch 10, umfassend mindestens 95 Gew.-% Phytosterolester und/oder Phytostanolester von hauptsächlich ungesättigten Fettsäuren.

12. Nahrungsergänzungsmittel oder Lebensmittelprodukt, umfassend die Stoffzusammensetzung nach einem der Ansprüche 1 bis 11.

13. Verfahren zur Herstellung einer Stoffzusammensetzung, umfassend Diacylglycerin(e), die hauptsächlich 1,3-Diacylglycerin(e) und mindestens ein in Fischöl gelöstes oder dispergiertes Phytosterolester und/oder Phytostanolester sind, wobei das Verfahren den Schritt des Veresterns von Phytosterol(en) und/oder Phytostanol(en) in Fischöl zum Ergeben eines Produkts umfasst (bezeichnet als Mischung A), das aus mindestens einem Phytosterolester und/oder Phytostanolester besteht, und Diacylglycerin(en), die hauptsächlich 1,3-Diacylglycerin(e) in Fischöl sind.

14. Verfahren nach Anspruch 13, wobei das Produkt in zwei sichtbar verschiedene Fraktionen trennbar ist oder diese umfasst, eine Ölfraktion, umfassend Diacylglycerin(e), die hauptsächlich 1,3-Diacylglycerin(e), Triacylglycerin(e) und Phystosterolester und/oder Phytostanolester von hauptsächlich ungesättigter/ungesättigten Fettsäure(n) sind, und eine pastenartige Fraktion, umfassend Diacylglycerin(e), die hauptsächlich 1,3-Diacylglycerin(e), Triacylglycerin(e) und Phystosterolester und/oder Phytostanolester von hauptsächlich gesättigter/gesättigten Fettsäure(n) sind.

15. Verfahren nach Anspruch 13, wobei die Ölfraktion etwa 10 bis 20 Gew.-% Phytosterolester und/oder Phytostanolester von hauptsächlich ungesättigter/ungesättigten Fettsäure(n) enthält und die pastenartige Fraktion des Produkts etwa 30 bis 35 Gew.-% Phytosterolester und/oder Phytostanolester aus hauptsächlich gesättigter/gesättigten Fettsäure(n) enthält.

16. Verfahren nach einem der Ansprüche 13 oder 15, wobei das oder die Phytosterolester und/oder das oder die Phytostanolester C₁₄-C₂₂-Ester sind, vorzugsweise gesättigte oder ungesättigte C₁₆-C₁₈-Fettsäuren, wobei die Fettsäuren ausgewählt sind aus der Gruppe, bestehend aus Öl-, Linol-, Linolen-, Palmitin- und Stearinsäuren.

17. Verfahren nach einem der Ansprüche 13 bis 16, wobei das Phytosterol ausgewählt ist aus der Gruppe, bestehend aus beta-Sitosterol, Campesterol, Stigmasterol, Brassicasterol, und wobei das Phytostanol ausgewählt ist aus der Gruppe, bestehend aus Campestanol und Sitostanol.

18. Verfahren nach einem der Ansprüche 13 bis 17, ferner umfassend die Schritte des Trennens der beiden Fraktionen von Mischung A, um ein erstes Produkt (bezeichnet als Mischung B) zu ergeben, das Diacylglycerin(e), die hauptsächlich 1,3-Diacylglycerin(e), Triacylglycerin(e) und Phytosterolester und/oder Phytostanolester von hauptsächlich gesättigten Fettsäuren sind, und ein zweites Produkt (bezeichnet als Mischung C), das Diacylglycerin(e), die hauptsächlich 1,3-Diacylglycerin(e), Triacylglycerin(e) und Phytosterolester und/oder Phytostanolester von hauptsächlich ungesättigten Fettsäuren sind, umfasst.

19. Verfahren nach Anspruch 18, wobei die Trennung der zwei Fraktionen der Mischung A durch Zentrifugieren erfolgt.

20. Verfahren nach Anspruch 18, wobei der Gehalt an Phytosterolester(n) und/oder Phytostanolestern in der Mischung C ferner zum Ergeben eines Produkts (bezeichnet als Mischung C1) angereichert wird, das mindestens 25 Gew.-% Phytosterol- und/oder Phytostanolester von hauptsächlich ungesättigten Fettsäuren umfasst.

21. Verfahren nach Anspruch 20, wobei die Mischung C durch Behandlung mit Aceton angereichert ist.

22. Verfahren nach Anspruch 20, wobei die Mischung C durch Behandlung mit Toluol angereichert ist, um reine Phytosterol- und/oder Phytostanolester (mindestens 95 Gew.-%) von hauptsächlich ungesättigten Fettsäuren (bezeichnet als Mischung C2) zu ergeben.

23. Verwendung einer Stoffzusammensetzung, die eine Mischung aus Diacylglycerin(en) umfasst, die hauptsächlich 1,3-Diacylglycerin(e) und Triacylglycerin(e) mit Phytosterolester und/oder Phytostanolester von hauptsächlich in Fischöl gelöster/gelösten oder dispergierter/dispergierten ungesättigter/ungesättigten Fettsäure(n) sind, als Nährstoff oder Nahrungsergänzungsmittel.

24. Nährstoff oder Nahrungsergänzungsmittel nach Anspruch 12, ferner umfassend Monoacylglycerin(e).

25. Nährstoff oder Nahrungsergänzungsmittel nach Anspruch 12 oder Anspruch 24 in Dosierungseinheitsform, vorzugsweise als Tablette oder Kapsel.

26. Nährstoff nach einem der Ansprüche 12, 24 und 25, der ein beliebiges von cholesterinarmer Butter, Kakaobutter, wasserfreiem Milchfett, Eis, Kaffeeweißer und Sahne, Milchprodukten, insbesondere Käse und anderen cholesterinhaltigen Lebensmitteln ist.

## Revendications

1. Composition de matière comprenant un/des diacylglycérol(s) qui est/sont principalement un/des 1,3-diacylglycérol(s) et au moins un ester de phytostérol et/ou un ester de phytostanol dissous ou dispersé(s) dans l'huile de poisson.

2. Composition de matière selon la revendication 1, dans laquelle ladite composition peut être séparée en deux fractions visiblement distinctes qui sont une fraction d'huile comprenant un/des diacylglycérol(s) qui est/sont principalement un/des 1,3-diacylglycérol(s), un/des triacylglycérol(s) et des esters de phytostérol et/ou des esters de phytostanol d'acide(s) gras principalement insaturé(s) et une fraction pâteuse comprenant un/des diacylglycérol(s) qui est/sont principalement un/des 1,3-diacylglycérol(s), un/des triacylglycérol(s) et des esters de phytostérol et/ou des esters de phytostanol d'acide(s) gras principalement saturé(s).

3. Composition de matière selon la revendication 1, dans laquelle ladite composition comprend deux fractions distinctes qui sont une fraction d'huile comprenant un/des diacylglycérol(s) qui est/sont principalement un/des 1,3-diacylglycérol(s), un/des triacylglycérol(s) et un/des ester(s) de phytostérol et/ou un/des ester(s) de phytostanol d'acide(s) gras principalement insaturé(s) et une fraction pâteuse comprenant un/des diacylglycérol(s) qui est/sont principalement un/des 1,3-diacylglycérol(s), un/des triacylglycérol(s) et un/des ester(s) de phytostérol et/ou un/des ester(s) de phytostanol d'acide(s) gras principalement saturé(s).

4. Composition de matière selon l'une quelconque des revendications 1 à 3, comprenant 1 à 99% en poids de diacylglycérol(s) qui est/sont principalement un/des 1,3-diacylglycérol(s) et 1 à 99% en poids dudit/desdits ester(s) de phytostérol et/ou ester(s) de phytostanol dissous ou dispersé(s) dans l'huile de poisson.

5. Composition de matière selon l'une quelconque des revendications 1 à 4, comprenant en outre des monoglycérides.

6. Composition de matière selon la revendication 4 ou 5, comprenant 1 à 99% en poids de diacylglycérol(s) qui est/sont principalement des 1,3-diacylglycérols, 1 à 99% en poids d'ester(s) de phytostérol et/ou d'ester(s) de phytostanol et 0 à 50% en poids de monoglycérides et 1 à 99% en poids de triacylglycérol(s), de préférence ladite composition comprend 3 à 50% en poids de diacylglycérol(s) qui est/sont principalement des 1,3-diacylglycérols, 7 à 48% en poids d'ester(s) de phytostérol et/ou d'ester(s) de phytostanol et 2 à 90% en poids de triacylglycérol(s).

7. Composition de matière selon l'une quelconque des revendications 1 à 6, dans laquelle ledit phytostérol est choisi dans le groupe constitué du bêta-sitostérol, du campestérol, du stigmastérol et du brassicastérol, et dans laquelle le phytostanol est choisi dans le groupe constitué du stigmastanol, du campestanol et du sitostanol.

8. Composition de matière selon l'une quelconque des revendications 1 à 7, dans laquelle ledit/lesdits ester(s) de phytostérol et/ou esters de phytostanol est/sont des esters d'acides gras saturés ou insaturés en C₁₄ à C₂₂, de préférence en C₁₆ à C₁₈, comme par exemple les acides oléique, linoléique, linolénique, palmitique et stéarique.

9. Composition de matière selon la revendication 8, dans laquelle ledit/lesdits ester(s) de phytostérol et/ou esters de phytostanol est/sont des esters d'acides gras insaturés en C₁₄ à C₂₂, de préférence en C₁₆ à C₁₈.

10. Composition de matière selon la revendication 1, qui comprend un/des diacylglycérol(s) qui est/sont principalement un/des 1,3-diacylglycérol(s), un/des triacylglycérol(s) et au moins 25% en poids d'ester(s) de phytostérol et/ou d'ester(s) de phytostanol d'acides gras principalement insaturés.

11. Composition selon la revendication 10, comprenant au moins 95% en poids d'ester(s) de phytostérol et/ou d'ester(s) de phytostanol d'acides gras principalement insaturés.

12. Complément alimentaire diététique ou produit alimentaire comprenant la composition de matière selon l'une quelconque des revendications 1 à 11.

13. Procédé pour préparer une composition de matière comprenant un/des diacylglycérol(s) qui est/sont principalement un/des 1,3-diacylglycérol(s) et au moins un/des ester(s) de phytostérol et/ou un/des ester(s) de phytostanol, dissous ou dispersé(s) dans l'huile de poisson, ledit procédé comprenant l'étape qui consiste à estérifier le/les phytostérol(s) et/ou phytostanol(s) dans l'huile de poisson pour donner un produit (appelé Mélange A) constitué d'au moins un ester de phytostérol et/ou un ester de phytostanol, et de diacylglycérol(s) qui est/sont principalement un/des 1,3-diacylglycérol(s) dans l'huile de poisson.

14. Procédé selon la revendication 13, dans lequel ledit produit peut être séparé en ou comprend deux fractions visiblement distinctes, qui sont une fraction d'huile comprenant un/des diacylglycérol(s) qui est/sont principalement un/des 1,3-diacylglycérol(s), un/des triacylglycérol(s) et des esters de phytostérol et/ou des esters de phytostanol d'acide(s) gras principalement insaturé(s) et une fraction pâteuse comprenant un/des diacylglycérol(s) qui est/sont principalement un/des 1,3-diacylglycérol(s), un/des triacylglycérol(s) et des esters de phytostérol et/ou des esters de phytostanol d'acide(s) gras principalement saturé(s).

15. Procédé selon la revendication 13, dans lequel ladite fraction d'huile contient environ 10 à 20% en poids d'ester(s) de phytostérol et/ou d'ester(s) de phytostanol d'acide(s) gras essentiellement insaturé(s) et ladite fraction pâteuse dudit produit contient environ 30 à 35% en poids d'ester(s) de phytostérol et/ou d'ester(s) de phytostanol d'acide(s) gras principalement saturé(s).

16. Procédé selon l'une quelconque des revendications 13 et 15, dans lequel lesdits esters de phytostérol et/ou esters de phytostanol sont des esters d'acides gras saturés ou insaturés en C₁₄ à C₂₂, de préférence en C₁₆ à C₁₈, où lesdits acides gras sont choisis dans le groupe constitué des acides oléique, linoléique, linolénique, palmitique et stéarique.

17. Procédé selon l'une quelconque des revendications 13 à 16, dans lequel ledit phytostérol est choisi dans le groupe constitué du bêta-sitostérol, du campestérol, du stigmastérol, du brassicastérol, et ledit phytostanol est choisi dans le groupe constitué du campestanol et du sitostanol.

18. Procédé selon l'une quelconque des revendications 13 à 17, comprenant en outre les étapes qui consistent à séparer les deux fractions dudit mélange A, pour donner un premier produit (appelé Mélange B) qui comprend un/des diacylglycérol(s) qui est/sont principalement un/des 1,3-diacylglycérol(s), un/des triacylglycérol(s) et un/des ester(s) de phytostérol et/ou un/des ester(s) de phytostanol d'acides gras principalement saturés et un deuxième produit (appelé Mélange C) qui comprend un/des diacylglycérol(s) qui est/sont principalement un/des 1,3-diacylglycérol(s), un/des triacylglycérol(s) et un/des ester(s) de phytostérol et/ou un/des ester(s) de phytostanol d'acides gras principalement insaturés.

19. Procédé selon la revendication 18, dans lequel la séparation des deux fractions du Mélange A est effectuée par centrifugation.

20. Procédé selon la revendication 18, dans lequel la teneur en ester(s) de phytostérol et/ou en esters de phytostanol dans ledit Mélange C est en outre enrichie pour donner un produit (appelé Mélange C1) comprenant au moins 25% en poids d'esters de phytostérol et/ou de phytostanol d'acides gras principalement insaturés.

21. Procédé selon la revendication 20, dans lequel ledit mélange C est enrichi par un traitement avec de l'acétone.

22. Procédé selon la revendication 20, dans lequel ledit Mélange C est enrichi par un traitement avec du toluène, pour donner des esters purs de phytostérol et/ou de phytostanol (au moins 95% en poids) d'acides gras principalement insaturés (appelé Mélange C2).

23. Utilisation d'une composition de matière qui comprend un mélange de diacylglycérol(s) qui est/sont principalement un/des 1,3-diacylglycérol(s) et de triacylglycérol(s) avec un/des ester(s) de phytostérol et/ou un/des ester(s) de phytostanol d'acide(s) gras principalement insaturé(s) dissous ou dispersé(s) dans l'huile de poisson, comme nutriment diététique ou complément alimentaire.

24. Nutriment diététique ou complément alimentaire selon la revendication 12, comprenant en outre un/des monoacylglycérol(s).

25. Nutriment diététique ou complément alimentaire selon la revendication 12 ou 24, sous forme de dosage unitaire, de préférence, un comprimé ou une capsule.

26. Nutriment diététique selon l'une quelconque des revendications 12, 24 et 25, qui est un élément quelconque parmi du beurre à faible teneur en cholestérol, du beurre de cacao, la matière grasse anhydre du lait, une crème glacée, un colorant et une crème à café, un produit laitier, en particulier du fromage et d'autres aliments contenant du cholestérol.
